(19) Europäisches Patentamt
European Patent Office
Office européen des brevets

(11) **EP 2 492 340 B1**

(12) **EUROPEAN PATENT SPECIFICATION**

(45) Date of publication and mention
of the grant of the patent:
**22.04.2015  Bulletin 2015/17**

(51) Int Cl.:
***C12N 5/00*** *(2006.01)*

(21) Application number: **11305122.1**

(22) Date of filing: **07.02.2011**

(54) **SOFT SUBSTRATE FOR CELL CULTURE AND PROCESS FOR PREPARING THE SAME**

WEICHES SUBSTRAT FÜR ZELLKULTUR UND VERFAHREN ZU DESSEN HERSTELLUNG

SUBSTRAT MOU POUR CULTURE CELLULAIRE ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**29.08.2012  Bulletin 2012/35**

(73) Proprietor: **Commissariat à l'Énergie Atomique et aux Énergies Alternatives
75015 Paris (FR)**

(72) Inventors:
• **Tseng, Qingzong
New Taipei City 222 (TW)**
• **Thery, Manuel
38000 Grenoble (FR)**

(74) Representative: **Tezier Herman, Béatrice et al
Cabinet Becker & Associés
25, rue Louis le Grand
75002 Paris (FR)**

(56) References cited:
**WO-A2-2010/011407**

• **AMMAR AZIOUNE ET AL: "Simple and rapid process for single cell micro-patterning", LAB ON A CHIP, vol. 9, no. 11, 1 January 2009 (2009-01-01), page 1640, XP55010849, ISSN: 1473-0197, DOI: 10.1039/b821581m**

• **ALEXANDER WELLE ET AL: "UV-Based Patterning of Polymeric Substrates for Cell Culture Applications", BIOMEDICAL MICRODEVICES, KLUWER ACADEMIC PUBLISHERS, BO, vol. 4, no. 1, 1 March 2002 (2002-03-01), pages 33-41, XP019205055, ISSN: 1572-8781, DOI: 10.1023/A:1014267712144**

• **MITCHELL S A ET AL: "Cellular attachment and spatial control of cells using micro-patterned ultra-violet/Ozone treatment in serum enriched media", BIOMATERIALS, ELSEVIER SCIENCE PUBLISHERS BV., BARKING, GB, vol. 25, no. 18, 1 August 2004 (2004-08-01), pages 4079-4086, XP004497070, ISSN: 0142-9612, DOI: 10.1016/J.BIOMATERIALS.2003.11.010**

• **VESNA DAMLJANOVIC ET AL: "Bulk and micropatterned conjugation of extracellular matrix proteins to characterized polyacrylamide substrates for cell mechanotransduction assays", BIOTECHNIQUES, vol. 39, no. 6, 1 December 2005 (2005-12-01), pages 847-851, XP55010845, ISSN: 0736-6205, DOI: 10.2144/000112026**

• **PETERBAUER THOMAS ET AL: "Simple and versatile methods for the fabrication of arrays of live mammalian cells", LAB ON A CHIP, ROYAL SOCIETY OF CHEMISTRY, CAMBRIDGE, GB, vol. 6, no. 7, 1 January 2006 (2006-01-01) , pages 857-863, XP002563077, ISSN: 1473-0197, DOI: 10.1039/B601803C [retrieved on 2006-05-08]**

• **QINGZONG TSENG ET AL: "A new micropatterning method of soft substrates reveals that different tumorigenic signals can promote or reduce cell contraction levels", LAB ON A CHIP, vol. 11, no. 13, 1 January 2011 (2011-01-01), page 2231, XP55010794, ISSN: 1473-0197, DOI: 10.1039/c0lc00641f**

## Description

## Field of the Invention

The present invention relates to a method for producing a soft substrate and to the soft substrate produced thereby, in particular a micropatterned soft substrate, said substrate being particularly useful for cell culture.

## Background of the Invention

Geometrical and mechanical properties of cell microenvironment have profound impact on cell morphogenesis and functions. They will affect cell cytoskeleton architecture, polarity, migration, division, growth, and differentiation. Biomaterials have been engineered to reveal these effects, investigate the mechanisms by which they regulate cell functions and eventually control them to design new tools for tissue engineering applications.

Microenvironment geometry has been controlled with micro-patterning techniques. They were used to control the localization of adhesive molecules from the extra-cellular matrix (ECM) and thereby control individual cell position and shape (Damljanovic V, et al., Biotechniques 2005; 39, 847-851). Cell shape control using adhesive micropattern is an efficient method. Indeed, adhesive micropatterns coated with ECM allow the normalization of individual cell shape and an accurate control of the spatial distribution of focal adhesion and actin cables (Parker et al., 2002, FASEB J 16, 1195-1204; Thery et al., 2006, Cell Motil Cytoskeleton 63, 341-355; Thery et al., 2006, Proc Natl Acad Sci USA 103, 19771-19776). Appropriate geometries can impose stringent orientation constraints to actin assembly, and reduce cell-cell variability.

Microenvironment mechanics have also been controlled with soft substrates. Both parameters, geometry and mechanics, should be controlled on the same material to faithfully recapitulate physiological conditions cells encounter in tissue and fully control the physical signals they are sensitive to. Cell shape control on deformable substrates has been performed using various micro-fabricated tools on either poly-acrylamide (PA) gels or arrays of micro-pillars. Micro-molded stamps (Damljanovic V, et al., *supra),* stencils (Parker et al., 2002, FASEB J 16, 1195-1204; Wang et al., 2002, Cell Motil Cytoskeleton 52, 91-106) or micro fluidic channels (Engler AJ, et al., 2004, J Cell Biol, 166, 877-887) were used to deposit locally ECM proteins on chemically activated PA. Micro-contact printing was used to print ECM proteins on arrays of micro-pillars (Liu et al, 2002, FASEB J, 16, 1195-1204; Ruiz et al, 2008, Stem Cells, 26, 2921-2927; Tan et al, 2003, Proc Natl Acad Sci USA, 100, 1484-1489; Fu et al, 2010, Nat Methods, 7, 733-736). WO 2010/011407 also discloses methods of generating patterned soft substrates.

Microenvironment geometry and mechanics affect cell architecture and function notably by modulating cell production of forces. Cells attach to their microenvironment and exert traction forces via the myosin dependent contraction of their actin cytoskeleton. In spatially confined, non-migrating, cells, stress fibers form along cell edges and traction forces are applied at cell apices. Stress fibers appear stronger when no ECM is available for cell adhesion in between cell apices. Microenvironment stiffness will affect the magnitude of forces: the stiffer the substrate, the larger the traction forces. The level of orientation of traction forces affects intra-cellular organization, notably nucleus positioning, centrosome positioning, primary cilum growth and intra-cellular trafficking. It is therefore a major regulator of cell polarity. Cell contractility also governs extensive cytoskeletal remodeling during cell migration, and division. Cell contractility not only regulates cell architecture and internal organization but also affects cell fate. It promotes cell growth and directs stem cell differentiation. Considering this broad impact of actin cytoskeleton contractility on cell physiology, it is not surprising that misregulation of force production are involved in tumoral transformation. Polarity misorientation, disordered cell positioning and amplified growth are characteristic tumoral features that can be induced by elevated cell contractility.

Two main methods to measure cell tractions forces are based on cell culture substrate deformation. However, they both have limitations in substrate fabrication and force analysis.

Among different methods being developed for force measurement, Traction Force Microscopy (TFM) is one of the most used. However, due to its rather complicate data processing step, this technique still remains exclusive to some specialized groups. Albeit all the materials needed to perform TFM are nearly routine equipments and reagents in ordinary biological laboratory.

The classical TFM proposed by Dembo and Wang (1999, Biophys J 76, 2307-2316)) was done on poly-acrylamide (PAA) gel. Basically, the acrylamide and N,N-methylene-bis-acrylamide solution was prepared with fluorescent micro-beads added to the solution. Then, the gel formed on an activated coverslip was activated by a bifunctional reagent, Sulfo-SANPAH, and UV light exposition. The gel was then coated homogeneously with extra-cellular matrix (ECM) protein to make the gel available for cell adhesion. When cells attached to the gel, due to the traction force exerted by the cell, the soft substrate deformed and thus the beads displaced. By comparing the image of the displaced beads and another image of the original beads position taken after detaching the cell (e.g., by trypsin treatment), one can obtain the displacement field. The traction force could therefore be obtained by solving a displacement-force inverse problem.

PAA gel activation with sulfo-SANPAH is a quite variable step resulting in non homogeneous and non reproducible activation of the substrate.

A second method, cell culture on micro-fabricated pillars allows a much simpler and thus faster force calculation (du Roure et al., 2005, Proc Natl Acad Sci USA 102, 2390-2395; Tan et al., 2003, Proc Natl Acad Sci USA 100, 1484-1489). However the substrate requires several non-trivial microfabrication steps. In addition, micropillars do not support solvent dewetting, and substrate topography can affect cell behavior. Micropatterning on PAA gel has been realized with stencils (Parker et al., 2002, *supra*; Wang et al., 2002, Cell Motil Cytoskeleton 52, 91-106), or micro structured stamps (Engler et al., 2004, Cell 126, 677-689; Tan et al., 2003, *supra*) but micropattern resolution is relatively low. In both cases, micropatterning requires several microfabrication steps, making the whole process long and difficult to realize. Accordingly, the development of substrate with adjustable softness, in particular micropatterned substrate, constitutes a major step towards the fabrication of a controlled microenvironment that is highly similar to *in situ* conditions.

Therefore, there exists still a need in the art for methods for producing soft gels that can be differentially functionalized, micropatterned and homogeneously activated, and can be easily produced with an enhanced reproducibility and resolution.

The inventors developed a new, fast, efficient and robust micropatterning method on soft substrate in which no microfabricated tool was required except the commercially available photomask. The method has been validated by controlling the shape, cytoskeleton architecture and traction force production of human mammary epithelial cells.

**Summary of the Invention**

The main objective of this invention is to provide new soft substrates that allow cell culture, in particular cell culture on soft micropatterned substrates. Soft substrates of the invention present a higher spatial resolution and allow a better absorption of biomolecules, such as proteins, than currently used substrates. Further, the process for producing soft substrates of the invention is simpler and faster than already described processes as it involves less microfabrication steps.

In this invention, the inventors designed a new process for preparing soft substrates particularly useful for cell culture, involving a key step of *in situ* gel polymerization on a mask.

**Brief Description of the Drawings**

Figure 1: Comparison of a polyacrylamide gel patterned with fluorescent fibronectin according to the invention (Fig. 1A) and a gel polymerized before being put into contact with the mask for UV exposure (Fig 1B). Scale bar represents 30μm.

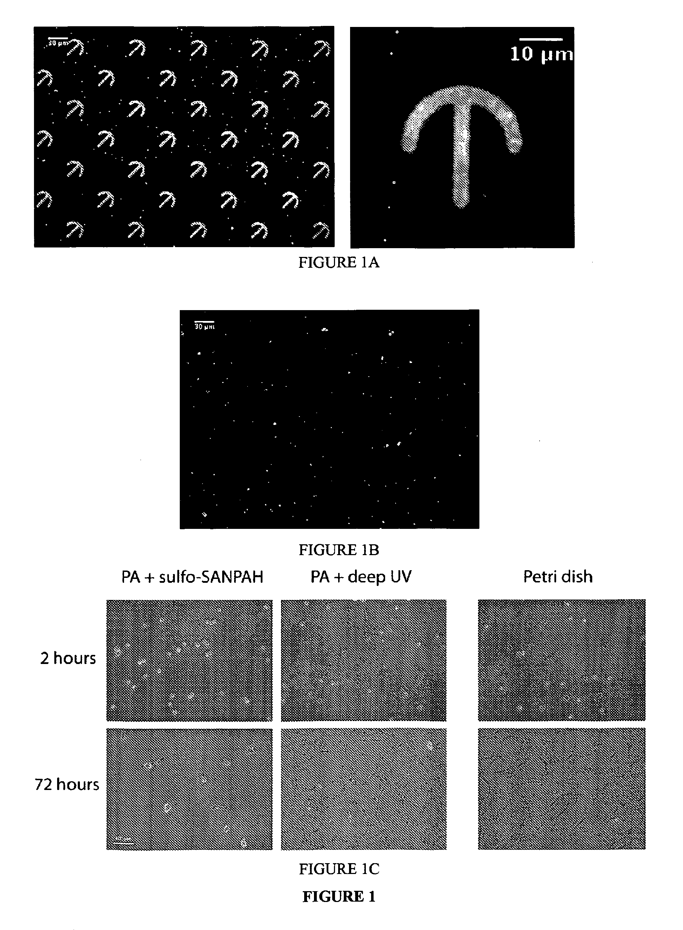

In Figure 1C, Cells have been trypsinized and plated on PA gels activated with the sulfo-SANPAH activation method on PA gels activated with deep UV and on Petri dish (made of plasma treated polystyrene) as a control. Pictures were taken 2 hours as well as 72 hours after cell plating. While cells have only started to attach on sulfo-SANPAH treated PA after 2h, most of them have started or completed their spreading phase on deep UV treated PA or Petri dish. 72 hours after plating, few cells have spread locally on sulfo-SANPAH treated PA but did not proliferate, while they have proliferated all over the deep UV treated PA or Petri dish.

Figure 2: Micropatterning of PAA gel.

Figure 2A: PAA micropatterning method. The gel is polymerized on the photomask, exposed to deep UV and coated with ECM proteins. Cells attach specifically to the UV exposed regions.

Figure 2B: Fibronectin and fibrinogen-A546 coating on micropatterned PAA. Scale bar represents 10 μm.

Figure 2C: Fibronectin and fibrinogen-A546 coating on micropatterned PAA. Scale bar represents 500 μm.

Figure 2D: MCF10A cells (phase contrast) plated on crossbow shaped micropattern (red) on PAA. Cells specifically attach and on micropatterns. Scale bar represents 100 μm.

Figure 3: Actin cytoskeleton streamlining normalizes cell traction force field

Figure 3A: Micropattern geometry orients actin network architecture. Individual MCF10A cells plated either on non-patterned, fibronectin coated, glass slide, or on disc, or pacman or crossbow shaped fibronectin micropatterns. Cells were fixed and stained with phalloidin to reveal F-actin filaments. Cells form preferentially contractile F-actin bundles, or stress fibers, above non-adhesive regions.

Figure 3B: Gel embedded beads were used to calculate cell traction force with Fourier transform traction cytometry. Pictures of beads were taken before and after cell detachment with trypsin to visualize gel deformation upon cell traction forces. Bead displacement were automatically detected and processed to infer the corresponding traction force field (see Materials and Methods). The right picture is a zoom picture of the squared zone shown in the left picture.

Figure 3C: Traction force field calculation shows that cell exhibit unpredictable stress spatial distribution in non-patterned and in disc-shaped patterned cells. Cells patterned on pacman, and crossbow, develop enhanced traction forces on adhesion sites flanking non-adhesive regions.

Figure 3D: Overlaying and averaging of traction force fields highlight the variability of traction force field in non patterned cells. Non patterned cells were aligned using their nucleus position. Force fields were more precisely quantified in micropatterned cells. Crossbow shaped micropatterns reproducibly concentrate the location of cell traction forces in the bottom part of the vertical bar.

Scale bar is 10 μm. Traction magnitude is given in Pascals.

**Figure 4**: Simple measurement of micropattern deformation allows easy, fast and accurate force quantification

Figure 4A: Fibrinogen-Alexa 546 coating was used to measure micropattern deformation. Pictures of micropatterns were taken before and after cell detachment with trypsin to visualize micropattern deformation upon cell traction forces.

Figure 4B: Drawings represent micropattern shape before and after cell detachment. The micropattern deformation length corresponded to the distance between the two arrows. Micropattern deformation was then plotted against the total traction force exerted by the cell. Data points were fitted with a linear regression (full line). On discs, micropattern deformation could not be predicted. It was measured along an arbitrary vertical axis. The correlation in this case between total traction and micropattern deformation was not good.

Figure 4C: When disc deformation was measured along the axis displaying the largest deformation, the correlation was better.

Figure 4D: On pacman shaped micropatterns the correlation was not good since the deformation was quite small and associated to large errors.

Figure 4E: On crossbow shaped micropatterns cell total traction force could be directly correlated to micropattern deformation with a small deviation from the linear fit. This calibration curve was used in the following experiments.

Figure 4F: New methodology to measure cell traction forces without bead displacement measurements or inverse problem calculation.

**Figure 5**: Applications of large-scale force measurements to tumoral transformation analysis

Figure 5A: Cell traction forces in response to blebbistatin calculated with method illustrated in figure 4F. Increasing drug concentrations were successively applied to 6 cells. Micropattern contours show a representative micropattern relaxation upon increasing drug concentrations. In the graph, cell maximal tractions in the absence of Blebbistatin were renormalized. Error bars represent the standard deviation. Data were fitted with a single exponential decay (full line) to calculate the IC50, ie drug concentration for half effect.

Figure 5B: Cell traction forces over time in response to 50μM of Blebbistatin calculated with method illustrated in figure 4F. Micropattern contours show a representative micropattern relaxation over time. Measurements were performed on a single cell. Data were fitted with a single exponential decay (full line).

Figure 5C: Cell traction forces calculated with method illustrated in figure 4F in mutant or drug treated MCF10A cells mimicking tumor transformation. MCF10A WT cells were compared to CK2b knockdown cells, ErbB2 inducible cells and TGFb1 treated cells. Comparison between two sets of measures were performed using a Student T test: two tailed, 95% interval confidence: *=P<0,05 **=P<0,01 ***=P<0,001.

## Detailed description of the invention

A first object of the present invention is a process for producing a soft substrate particularly useful for cell culture, preferably a micropatterned soft substrate, comprising the steps of:

(a) producing a polymer between a base layer and a transparency mask comprising at least one transparent area and optionally at least one non-transparent area,
(b) exposing the polymer to deep UV through the mask,
(c) detaching the mask from the polymer,
(d) contacting the polymer with a biomolecule, and
(e) optionally washing away the excess of biomolecule.

### *Description of the steps of the process:*

Step (a):

Step (a) comprises the production of a polymer between a base layer and a transparency mask.

The base layer may be any substrate appropriate to support the polymer. Preferably, the substrate may be formed of a rigid or semi-rigid material, such as plastic, metal, ceramic, glass or combinations thereof. Preferably, the material is convenient for confocal, optical and/or fluorescence microscopies. In the more preferred embodiment, the substrate is made of glass, preferably silanised glass. Typically, the substrate is flat. For example, a convenient substrate according to the present invention is a coverslip or a slide.

The produced polymer may be any synthetic biocompatible polymer, in particular gel-forming polymer. The polymerization may be performed by any means known by one of ordinary skill in the art by implementing polymerization reagents. For instance, the produced polymer according to step (a) may be a polyethylene glycol or polyacrylamide (PAA). It can also be a co-polymer formed with acrylamide and any other polymer, such as polyethylene glycol or polypyrrole.

According to the invention, the polymerization reagents are preferably added as a solution onto the mask and then the base layer is applied thereon, as to form a "sandwich" between the base layer and the mask. Polymerization then occurs and the formed polymer may present finely-tuned stiffness.

The polymer can be more particularly any acrylic acid-based hydrogel constructed by free radical polymerization, such as polyacrylamide, poly(N-isopropylacrylamide), or poly(2- hydroxyethyl methacrylate). The monomeric acrylamide may be cross-linked by any diacrylate group, such as ethyleneglycol dimethacrylate and 1,4-butanediol dimethacrylate, or preferably by N,N' methylenebisacrylamide. The stiffness of the polymerized acrylamide may be tuned by varying the ratio of the cross-linker to the acrylamide subunit. By way of example, when a polyacrylamide is prepared, by varying the relative amounts of monomeric acrylamide and bis acrylamide, the stiffness of the resulting polyacrylamide gel may be increased (by using a higher relative amount of bis acrylamide) or decreased (by using a lower relative amount of acrylamide) (ref: Yeung et al., Cell Motility and the Cytoskeleton 60:24-34, 2005).

In addition, the stiffness of the produced gel may be modified by co-polymerizing the acrylamide with other polymers, such as polypyrrole and polyethylene-glycol. The acrylamide may be co-polymerized with polyacetylene group such as polypyrrole and polyaniline to give rise to a conductive polymer.

For instance, a preferred weight ratio of monomeric acrylamide and bis acrylamide is in the range between 10:1 to 100:1, preferably between 20:1 to 60:1, more preferably between 30:1 to 50:1, in particular about 40:1.

In an embodiment, the polymer is polyacrylamide and the polymerization is performed by radical polymerization in presence of tetramethylethylenediamine (TEMED) and ammonium persulfate.

Thickness of the produced polymer (or produced gel) may vary in a large extent. The thickness ranges generally from about 20 to about 200 $\mu$m, the preferred thickness is between about 50 and about 100 $\mu$m.

In an embodiment, fiducial markers such as labelled beads, preferably fluorescently labeled beads, can be added during step (a). The markers are generally mixed with at least one of the polymerization reagents prior to polymerization. For instance, carboxylate modified polystyrene fluorescent beads may be used. The convenient micro-beads are well-known in the art (see for instance, Dembo and Wang, 1999, Biophys J 76, 2307-2316; Marganski et al., 2003, Methods Enzymol 361, 197-211)

As mentioned before, polymer may be produced on the transparency mask comprising at least one transparent area and optionally at least one non-transparent area. Transparent areas correspond to surfaces of the mask that are transparent to the deep UV light used in step (b). Transparent areas are made of any material that is transparent to the deep UV light used in step (b). For instance, the transparent areas of the mask may be made of quartz. The transparency mask (also simply named the mask in the present description) may also be defined by the possibility of coexistence of two types of areas: transparent and non-transparent areas. If non-transparent areas are present, they are made of any material that is opaque to the deep UV light used in step (b). For instance, the non-transparent areas of the mask may be made of chromium. According to this embodiment, the chromium layer defines the non-transparent areas.

Where the transparency mask comprises at least one transparent area and at least one non-transparent area, the mask may be a quartz plate coated with a chromium layer, where the chromium layer is absent on specific areas, defining thereby the transparent areas. In this particular embodiment, mask is a quartz plate partially coated with a chromium layer. In this particular embodiment, the mask comprises non transparent areas where the quartz plate is coated with a chromium layer and transparent areas where the quartz plate is uncoated with a chromium layer. The transparent areas define areas potentially activated by UV and therefore where adhesive patterns are desired. The mask may comprise transparent areas and non-transparent areas of any desired shape.

The transparent areas have the desired form for the pattern, in particular for the micropattern. Indeed, the shape of transparent areas will define the shape of the adhesive micropattern. The transparent area may have any desired shape and size.

In a particular embodiment, the size of the transparent area is between 100 to 4000 $\mu$m$^2$, more preferably between 200 to 2000 $\mu$m$^2$, still more preferably between 500 to 1500 $\mu$m$^2$. The area will depend on the number of cells to be adhered thereon and the size of the considered cells. Indeed, the area of the transparent area may correspond to the area of cell spread on the adhesive micropattern.

For instance, the shape of transparent areas may have, for instance, a geometric shape such as a square, circle, star, diamond, triangle, line, or combinations thereof. The transparent areas may also form straight or curve lines, parallel or branched. The one skilled in the art knows the advantage to certain micropatterns. For instance, particular adhesive micropatterns of interest are disclosed in the following documents: WO2005/026313, WO2010/046459, and for review, Thery, 2010, J Cell Science, 123;

For cell traction force measurement, the shape of transparent areas may have a crossbow shape, as illustrated in the examples.

Preferably, the mask presents several transparent areas, defining thereafter potential adhesive patterns disposed on the gel. Accordingly, the resulting soft substrate forms a cellular array. More particularly, said mask comprises at least 2 transparent areas, preferably at least 5, 10, 100, 1 000, 10 000, or 100 000 transparent areas. In a preferred embodiment, said mask comprises between 10 and 50 000 transparent areas/cm$^2$, more preferably between 5 000 and 15 000 transparent areas/cm$^2$, still more preferably about 10 000 transparent areas/cm$^2$. Preferably, the transparent areas are separated by at least about 10 $\mu$m, preferably by at least about 20, 30,

or 50 μm.

By "about" is intended the value more or less than 5 %.

In another embodiment, the mask comprises only transparent areas. The process of the invention then leads to the formation of a soft substrate covered with the biomolecule, without any pattern.

Ste (b):

Step (b) corresponds to the irradiation of the formed polymer through the mask with deep UV (ultraviolet). In the present invention, deep UV refers to UV radiation with a wavelength inferior to 200 nm, in particular inferior to 190 nm and more specifically equal to 180 nm.

Step (b) may be for instance performed in a UV/ozone cleaner.

UV irradiation triggers activation of the polymer gel in the areas of the polymer exposed to the UV irradiation through the transparent areas of the mask. Further to irradiation, in said areas, the polymer may become activated for at least one biomolecule. The polymer may alternatively become activated for functionalization with a coupling agent (or linker) that will make the functionalized polymer activated for at least one biomolecule.

One advantage of the use of deep UV radiation is that no photoinitiator is necessary to create free radicals.

Step (c):

Detachment of the mask may be performed by any means known by one of ordinary skill in the art. Removal of the mask may be simply performed by manual removal.

Step (d):

Contacting the polymer to the solution comprising a biomolecule may be performed by any means known by one of ordinary skill in the art.

In the present invention, the term "biomolecule" refers to any protein, carbohydrate, lipid, nucleic acid, or combination thereof such as a glycoprotein, a glycolipid or a proteolipid. In a preferred embodiment, the biomolecule can comprise or be made of any molecule that promotes cell attachment. These molecules are well known to those of ordinary skilled in the art and comprise antigens, antibodies, cell adhesion molecules such as cadherin or fragment thereof, extracellular matrix molecules such as laminin, fibronectin, vitronectin, collagen, synthetic peptides, carbohydrates and the like. Preferably, said adhesive patterns comprise extracellular matrix molecules, more preferably fibronectin. Preferably, the biomolecules are labeled, preferably fluorescently labeled, or are mixed with labeled molecules, preferably fluorescently labeled.

The biomolecule is preferably contacted with the polymer as a solution in an appropriate solvent. Among appropriate solvents may be cited water and buffers such as HEPES (4-(2-hydroxyethyl)-1-piperazineethanesulfonic acid). The solution may additionally comprise at least one other biomolecule, such as fibrinogen.

Step (d) may be preferably divided into two steps:

(d1) contacting the polymer with a coupling agent (or a linker), optionally in presence of a catalyst, and
(d2): contacting the functionalized polymer obtained after step (d1) with the biomolecule.

In the present invention, a "linker" or "coupling agent" refers to a molecule that may be associated with the polymer to link the polymer to another molecule, in particular the biomolecule of the invention.

The coupling agent may be a heterobifunctional crosslinker like EDC (1-ethyl-3-(3-dimethylaminopropyl) carbodiimide), or SMCC (Succinimidyl 4-[N-maleimidomethyl]cyclohexane-1-carboxylate) ; or a homobifunctional crosslinker like DSG (Disuccinimidyl glutarate). According to a preferred embodiment, the coupling agent is EDC.

The catalyst may be for instance NHS (N-hydroxysuccinimide).

Step (e):

The excess of biomolecule and optionally other chemical components of step (d) may be washed away, for instance with water or any other appropriate solvent, including alcoholic solvent, such as isopropanol.

The process may additionally comprise a step comprising detaching the polymer from the base layer. This step may be performed in particular after step (e).

Contrary to the processes implemented in the art to produce currently used soft substrates, this process does not include any intermediate between the mask and the gel.

The fabrication process implies generally the use of a mask to create a mold such as a stencil or a stamp, and then the application of the mold onto the polymer gel.

The presently claimed process proceeds through direct application of the polymer forming solution onto the mask. The absence of intermediate and the *in situ* polymerization according to the invention trigger a high spatial resolution. Thus, the contours of the patterns of biomolecule on the gel, corresponding to the transparent areas of the mask that have been affected by the UV irradiation, are precisely defined. Figure 1 compares the patterns of a gel obtained according to the invention to the patterns obtained by a classical technique not involving *in situ* polymerization, i.e. wherein the gel polymerization is performed prior to contact with the mask for UV exposure.

Further, as the process of the invention comprises less microfabrication steps than the previously described processes, it is faster and easier to implement.

The process of the invention finally provides soft substrates of better quality because it is obtained a better biomolecule adhesion on the polymer than previously described soft substrates. This is essentially due to a better contact between the gel and the mask, which in turns ensured the activation by deep UV. In particular, the activation and the biomolecule adhesion are more homogeneous and more reproducible than those of comparable previously described soft substrates.

The obtained soft substrate is a material that is deformable, in particular flexible, pliable, or malleable, when exposed to an external force. Other physical characteristics appropriate to soft substrates suitable for use in the process of the invention include linear elasticity and incompressibility.

Soft substrates according to the invention have a Young's modulus in the range of about 0.1 to about 100 kilopascal (kPa). The soft substrates of the present invention include those that may be tunable to the stiffness of physiological tissues with a Young's modulus of about 0.1 to about 100 kPa, preferably about 0.5 to about 50 kPa, more preferably about 1 to about 20 kPa, and still more preferably about 1 to about 10 kPa. It is intended that the one skilled in the art will adapt this value to the contractibility of the studied cells. For instance, for a cell with a high contraction capacity, less flexible soft substrate will be used. At the opposite, if the cell has a weak contraction capacity, the soft substrate will be very flexible.

Another object of the present invention is a soft substrate obtainable by the process according to the invention. In an embodiment, the soft substrate is obtained by the process according to the invention. In particular, the soft substrate comprises one or several micropatterns.

The soft substrates according to the invention may be used for cell culture on soft substrates, with an optional controlled geometry. The soft substrates according to the invention may also be used for measuring cell traction forces.

For cell culture or measuring cell traction forces, the soft substrate of the invention is exposed to at least one cell type for a period of time sufficient to allow the cell(s) to bind to the soft substrate.

Accordingly, the present invention relates to a method for culturing cells comprising providing a patterned soft substrate of the invention, exposing said substrate to at least one cell for a period of time sufficient to allow the cell(s) to bind to the soft substrate, and culturing said cell(s). The present invention relates to a use of the soft substrate according to the invention for cell culture. It also relates to the use of a patterned substrate of the invention for culturing cells.

In a particular embodiment, the present invention also relates to a tissue engineering method. In this method, cells are bound to the soft substrate, in particular to the micropattern and they are allowed to proliferate, to adopt an organisation defined by adhesive micropattern geometry, optionally to differentiate and/or to create a multicellular structure of interest.

The present invention further relates to a method for assaying a cellular activity or property of a cell comprising providing a patterned soft substrate of the invention, exposing said substrate to at least one cell for a period of time sufficient to allow the cell(s) to bind to the soft substrate, and measuring the cellular activity or property of said cell. The cellular activity of the cell may include the cell contractility, the cell motility, the cell migration, the cell growth, the cell division, the ability to complete cytokinesis, the cell interaction with other cells, the cell differentiation, the cell autophagy, the cell apoptosis and the like. The cellular property may include the organelles position or arrangement (Golgi apparatus, nucleus, mitochondria, centrosome), the cytoskeleton architecture, actin network, microtubule network, intermediate filament network, the cell polarity, the plasma membrane composition, the cell internal trafficking, the mitotic spindle orientation, the division plane positioning and the like.

The present invention further relates to a method for identifying a molecule modulating a cellular activity or property of a cell comprising providing a patterned soft substrate of the invention, exposing said substrate to least one cell for a period of time sufficient to allow the cell(s) to bind to the soft substrate, and measuring the cellular activity or property of said cell in presence and in absence of the molecule, and determining the effect of the molecule on the cellular activity or property, wherein a modulation of the cellular activity or property indicates that the molecule is capable of modulating the cellular activity or property of a cell.

The molecule may be of various origin, nature and composition. It may be any organic or inorganic substance, such as a lipid, peptide, polypeptide, nucleic acid, sugar, small molecule, chemical agents, drugs, etc., in isolated or in mixture with other substances. For instance, the test compound can be an antibody, an antisense oligonucleotide, or a siRNA. The molecule may be all or part of a combinatorial library of products, for instance.

By "modulating" or "modulate" is intended that the activity may be increased or stimulated, or be decreased or inhibited; or that the property is changed. For instance, when an organelle's location is concerned, the position of the organelle changes when cells are incubated with the test molecule.

For instance, the method may be useful for drug discovery, for testing toxicity of the candidate/test molecule on cells, for identifying molecules that modulate cellular activity or property, for identifying molecules useful for treating or preventing a disease, and the like. In particular, the disease may be a cancer.

Any kind of cells can be used in the present invention. Preferably, the cells are eukaryotic. Cells can be from animal, mammalian, or human. Cells can be for example fibroblasts, mesenchymal cells, endothelial and epithelial cells. Cells can also be muscle cells or nerve cells. Muscle cells include smooth muscle cells, striated muscle cells, or cardiac cells. Cells can also be stem cells such as embryonic stem cells (primary and cell lines), fetal cells (primary and cell lines), adult stem cells and induced pluripotent stem cells (iPS). Cells can be derived from a healthy or pathologic tissue or organism. Accordingly, cells can be normal or abnormal cells. The cells can be wild type or modified cells (physically or genetically altered cells). In a particular example, the cells can be tumor cells. For example, a gene can be inactivated and these methods allow the study of the inactivated gene.

This invention is further illustrated by the following examples which should not be construed as limiting. The content of all references cited throughout the specification is incorporated herein by reference.

**EXAMPLES**

Example 1:

Cell ability to exert traction forces on their microenvironment through the development of intra-cellular tension strongly impacts cell physiology and notably tumoral transformation. Current methods to measure cell traction forces rely on the deformation of soft substrates. Exact forces cannot be directly inferred from deformation since local deformation result from both local and distant force application sites. Accurate measurements require either long calculations or sophisticated microfabrication steps to numerically or physically separate force application sites.

The inventors developed a new method to associate the control of the spatial distribution of cell traction forces on adhesive micropatterns with force measurement on soft deformable substrates. Cells pull on the micropattern and, on appropriate geometries, contract micropattern length in a standardized fashion. After a calibration of the force-deformation relationship, cell traction forces could be rapidly and accurately quantified by a single micropattern picture acquisition and length measurement.

The inventors applied this method to mammary epithelial cells traction force measurements in various conditions mimicking specific tumoral transformations. They found that, contrary to the current view, all transformation phenotypes were not associated to increased level of cell contractility.

**Results**

New micropatterning method on soft substrate

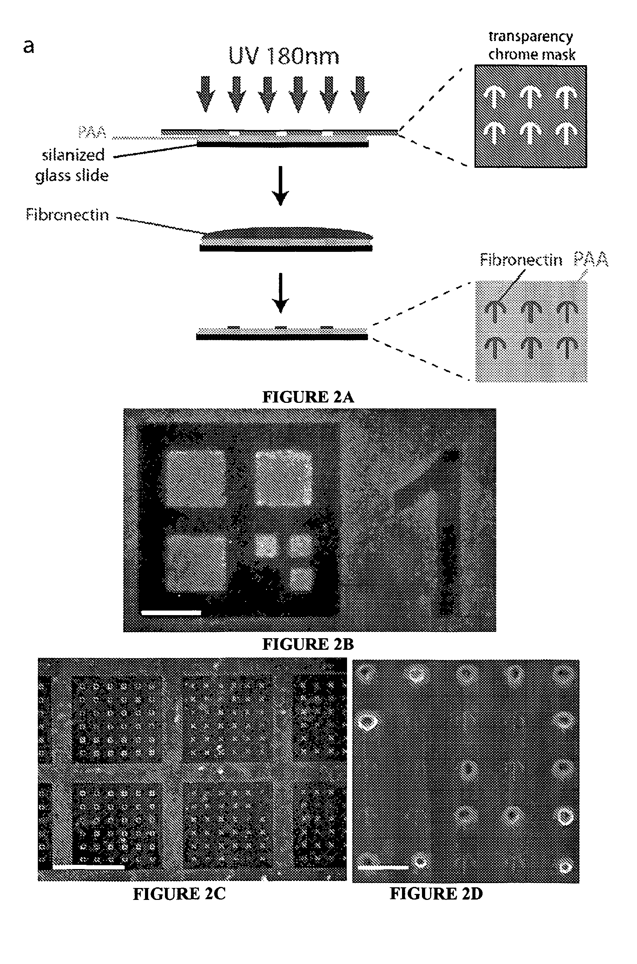

The inventors used direct exposure of PAA to deep UV through an optical mask to rapidly achieve micropatterning with high spatial resolution and reproducibility in order to precisely orient cell actin cytoskeleton in large-scale experiments (see Materials and Methods). A drop of acrylamide solution was placed directly on the chromium optical mask and covered with a silanized glass coverslip. After PAA polymerization, the sandwich was exposed to deep UV in order to oxidize the PAA through the micropatterned transparent regions of the optical mask (Fig. 2A). The coverslip along with the PAA gel was removed from the mask and coated with fibronectin, which adsorbed only on the UV-exposed regions. The direct contact with the optical mask during PAA polymerization and UV-exposure allowed a faithful reproduction of its spatial features and ensured a good, sub-cellular, spatial resolution (Fig. 2B). The coating was quite homogeneous over the entire coverslip (Fig. 2C). The entire process, from PAA preparation to the end of protein coating, lasts 2 hours, including 1 hour of PAA polymerization, and is highly robust.

Controled localisation and focusing of force application sites

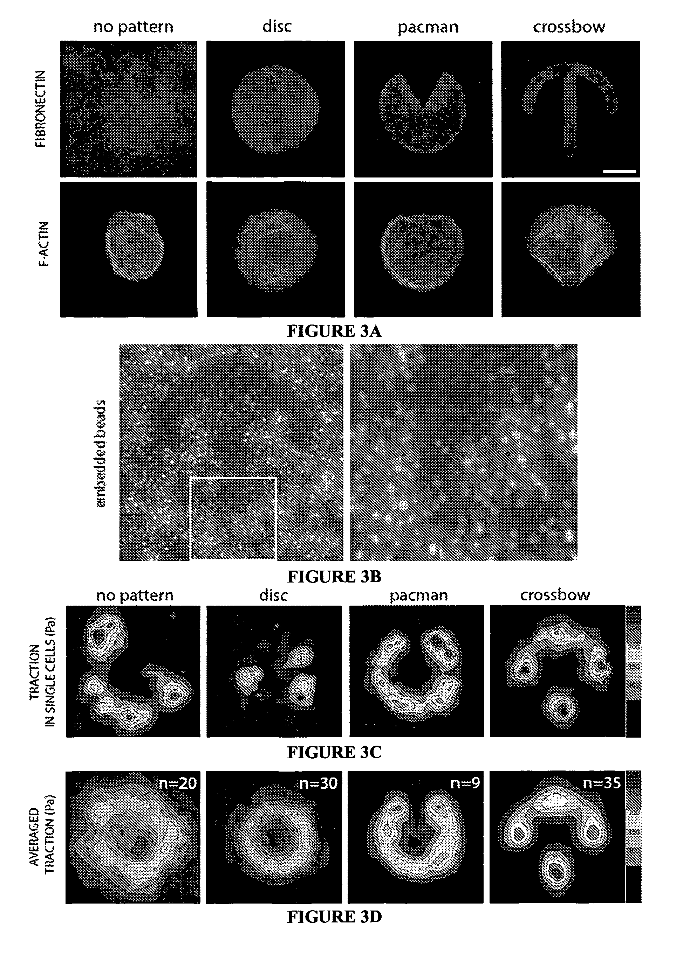

Non-tumorigenic human epithelial cells from the mammary gland, MCF10A, were plated on the micropatterned PAA substrates. Cells specifically attached to the fibronectin coated micropatterns since non-exposed PAA regions prevented protein adsorption (Fig. 2D). The effect of micropattern geometry on cell traction forces orientation was tested on various shapes: disc, pacman and crossbow (Fig. 3A). Spread cells exerted traction forces on the micropattern that could be measured by looking at the displacement of fluorescent beads embedded in the PAA gel (see Fig. 3B and Material and Methods). Particle Image Velocimetry followed by individual particle tracking were used to measure bead displacements ((Marganski et al (2003) Methods Enzymol 361:197-211; Sabass et al (2008) Biophys J 94(1):207-220). Force fields were calculated from the bead displacement fields by using the Fourier transform traction cytometry ((Sabass et al, *supra*; Butler et al (2002) Am J Physiol Cell Physiol 282(3):C595-605). Force fields exerted by individual cells were overlaid and averaged to quantify their reproducibility (Fig. 3C, D). On homogeneously coated regions cells developed forces that were randomly distributed from one cell to the other. In cells constrained on disc shaped micropatterns, forces were still randomly oriented but their magnitudes were lowered due to reduced cell spreading. In cells constrained on pacman shaped micropatterns, forces distribution was geometrically biased due to enhanced cell contraction above non adhesive regions. Cell ability to exert traction forces was even more stimulated on crossbow shaped micropatterns, where the total traction force per cell was higher than on any other micropattern shape (Fig. 3D). Importantly, most of the traction forces were reproducibly oriented upward, along the straight adhesive bar on the extremity of which the inventors measured the highest pressure (Fig. 3D). These results demonstrated that appropriate micropattern geometries can both stimulate cell contraction and orient force application. Such geometries place cells in convenient conditions to reveal cell potential contractility and measure their contraction strength.

Force field streamlining allows reproducible force-deformation relationship

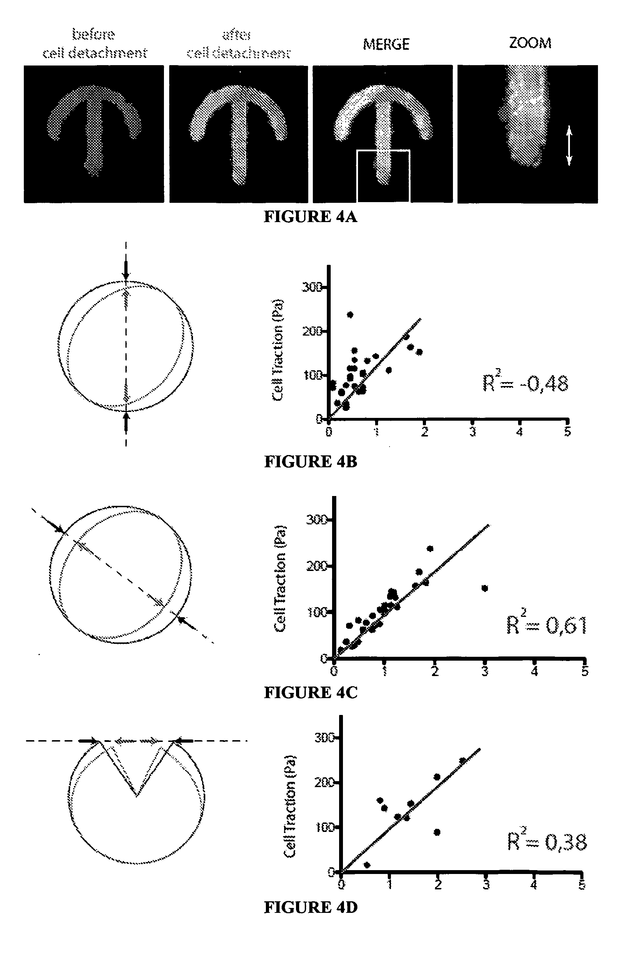

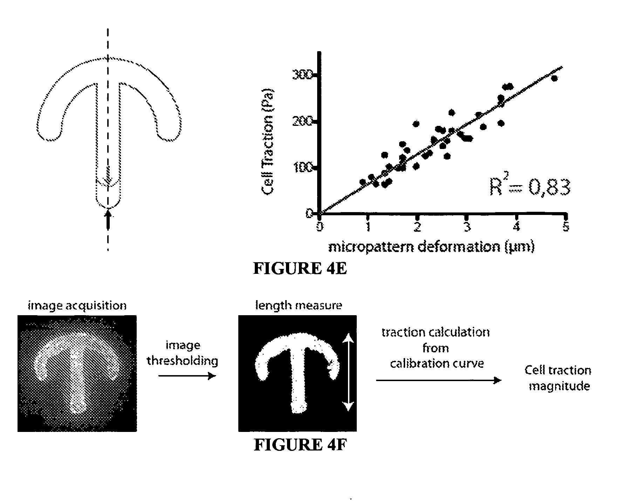

When actin cables have an unpredictable spatial distribution, a given deformation cannot be directly assigned to a defined force magnitude. Indeed, forces applied on adjacent adhesion sites both affect local deformations, due to the continuous nature of the substrate. Each deformation results from local and distant forces. So the calculation of the force field is highly dependent on the spatial distribution of force application sites and the relationship between deformation and forces varies from one cell to the other. The inventors hypothesized that in cells developing a reproducible architecture and a controlled pattern of force application, a given local deformation could be assigned to a defined value of the contractile force. To test this hypothesis, they used classical force field calculations with beads embedded in the PAA gel (Fig. 3B) and observation of micropattern deformation (Fig. 4A) to establish the force-deformation relationship in micropatterned cells. On disc, deformation orientation was unpredictable and no good correlation could be found between the deformation along a reference axis and the average traction force all over the cell (Fig. 4B). When the deformation was measured along the shortest, and therefore most contracted, cell axis, the correlation was improved (Fig. 4C). However this shape did not stimulate cell contraction and therefore did not fully reveal cell contractility potential (Fig. 3D). Cell traction force magnitudes were slightly higher on pacman shapes, but force and deformation were not precisely correlated (Fig. 4D). On the crossbow, the length of the straight bar is compressed in response to cell traction forces (Fig. 4A). The inventors found a good linear correlation between crossbow bar shortening, a local measure, and average cell traction, a global cell state (Fig. 4E). Each length variation could be assigned to a defined force value. This showed that crossbow bar length could be taken as a direct indicator of cell contraction level. On the crossbow, a single image acquisition was sufficient to measure the bar length and read the corresponding average cell traction force using the calibration curve (Fig. 5F). It was no longer necessary to measure embedded bead displacements or to perform long numerical calculations to solve the inverse problem and obtain the corresponding force value. Force measurement was not only easier and faster, compared to any previous method, it also became amenable to automation.

Validation of force measurement

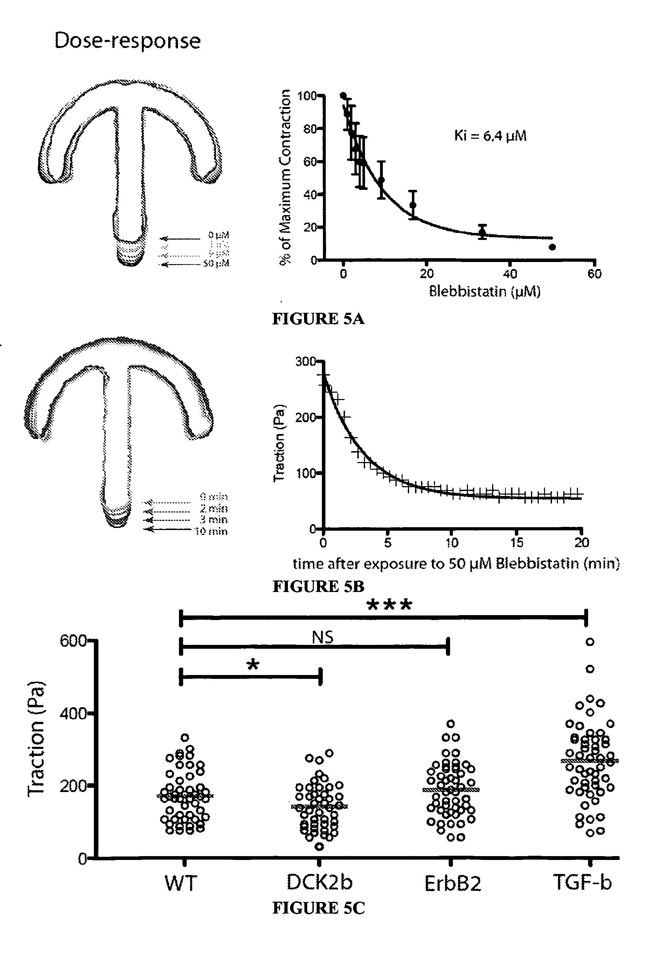

The inventors validated this methodology by analyzing the well-described Blebbistatin effects on cell contractility. Blebbistatin has been shown to inhibit myosin-II ATPase. Cells were treated with increasing dose of Blebbistatin. Crossbow bar length measures on thresholded pictures of fluorescent micropattern were used to measure cell traction forces (Fig. 5A). The force inhibition profile in response to increasing dose of blebbistatin matched the myosin II inhibition profile and the cellular force profile measured with other techniques (Mitrossilis D, et al. (2009) Proc Natl Acad Sci U S A 106(43):18243-18248). Drugs effects on cell contractility could thus easily and rapidly been quantified using this new methodology.

Another application requiring numerous, and thus fast, force measurements is the analysis of force relaxation over time. The inventors could also follow force magnitude decrease in response to $50\mu M$ of Blebbistatin and found that it follows a single exponential decay (Fig. 5B).

All tumorigenic transformation do not increase cell contractility

The inventors then used their method to compare the contraction level of wild type (WT) MCF10A cells to that of drug treated or genetically modified MCF10A cells mimicking some tumor transformation. Indeed tumor transformation has been shown to be associated with high levels of cell contraction. This suggested that cell contraction level measurements are required to understand the regulation of tumour progression and to develop improved treatments. Cell exposure to TGFb1 is known to induce epithelial to mesenchymal transition, which mimics some features of tumor transformation. TGFb1 treated cells are unable to form proper acinar structures when cultured on collagen gels. Instead they proliferate and form unstructured cell groups recapitulating tumour growth. MCF10A cells were treated for 2 days with 2ng/mL of TGFb1 before being plated on micropatterned PAA substrates. As expected, cells exhibited a significantly higher level of cell contraction as revealed by crossbow shortening (Fig. 5C). ErbB2 receptor activation is known to induce early stages of mammary carcinogenesis and prevent proper acinar structure formation in collagen gels. The inventors activated ErbB2 receptors with the ligand AP1510 in MCF10A expressing inducible ErbB2 receptor. Surprisingly, they found no significant changes in the level of cell contraction. Protein kinase CK2 (previously known as Casein Kinase 2) inactivation has also been shown to induce epithelial to mesenchymal transition and participate in tumor transformation and propagation. Interestingly, in CK2b knockdown cells, the contraction level was significantly lower than in WT cells (Fig. 5C). These results show that various types of tumorigenic transformation can either promote or reduce the level of cell contraction. They also demonstrate that the method of the inventors can easily be used at larger scale to characterize more precisely this complex correlation between cell contraction and tumoral transformation.

## Discussion

The use of deep UV exposure on PAA in contact with the photomask is to the inventors' knowledge the most robust and easiest method to create homogeneous and reproducible micropatterns on soft deformable substrates. The actin network streamlining and force field normalization in response to appropriate micropattern geometry allow a precise calibration of the relationship between micropattern shape deformation and traction forces. When force application sites are isolated from other sites by non-adhesive regions, the natural linear relationship between force and deformation of soft substrate is recovered, probably because deformation fields from each force application sites do not cross-over. Thanks to this linear relationship between force and deformation, force measurement is simply obtained by measuring micropattern length. Classical force measurement methods are still required to obtain the calibration curve. But, afterwards, a single image acquisition is sufficient to measure micropattern length and read the corresponding traction force. Therefore, force measurement is as simple as with the use of micropillars (Tan JL, et al. (2003) Proc Natl Acad Sci U S A 100(4):1484-1489) without the microfabrication constraint and issues of cells spreading in between the pillars. In addition, the inventors' method is easily amenable to automation since cell position and subcellular localization of force production are precisely controlled. This method paves the way to large scale and high throughput analysis of cell contraction state.

The initial work of the inventors identified tumorigenic transformations that were not associated to increased level of cell contractility. High levels of contractility were observed in epithelial cells forming disorganized multicellular structures or detaching from each others. Such phenotypes are characteristic of advanced or late stages of tumoral transformation. ErbB2 receptor activation is a feature of early tumoral transformation that impacts on growth rate. CK2 is also implicated in anti-apoptotic effect and CK2beta knockdown specifically affect p53-dependent cell survival. Although contractility activates cell growth, early phases of cancer progression involving cell growth stimulation might not systematically be associated to high level of contractility. It would now be necessary to analyze more specifically cell contraction level at various phases of tumoral transformation to clarify the relationship between cancer progression and cell contraction. Mechanical property characterization of healthy and transformed cells could then be used to set up a new medical diagnosis test.

## Materials and Methods

PAA micropatterning

25mm round glass coverslips were first cleaned with piranha for 2 hour and silanized by dipping in ethanol solution containing 2% (v/v)3-(Trimethoxysilyl)propyl methacrylate (Sigma) and 1% (v/v)acetic acid for 5min. After cleaning with ethanol to remove excess silane residue, the coverslips were incubated at 120°C for one hour.

Carboxylate modified polystyrene fluorescent beads (dark red 200nm, Invitrogen F-8807) were passivated by poly(ethylene)glycol as follow: fluorescent beads were diluted 25-fold in MES buffer (10mM pH 5.5) containing 8mg/mL N-hydroxysuccinimide (NHS; Fluka) and 4mg/mL EDC (1-Ethyl-3-[3-dimethylaminopropyl] carbodiimide Hydrochloride; Pierce) before 1:1 mixing with PLL-PEG (PLL(20)-g[3.5]-PEG(2); Susos) solution (4mg/mL in 10mM pH8.5 HEPES buffer). The mixture was incubated with rotation at 4°C overnight. The beads were subsequently spun down and resuspended in HEPES buffer (10mM pH7.4). Acrylamide solution containing 6.67% acrylamide and 0.167% bis-acrylamide was mixed with passivated fluorescent beads by sonication before additon of APS and TEMED. A drop of acrylamide solution was put directly on the chromium side of the photomask (Toppan). The photomask was cleaned by n-Hexane prior to use in order to maintain a hydrophobic surface. A silanized coverslip was placed over the drop and let it polymerize for 45 minutes. The sandwich was then exposed to deep UV in a UV/Ozone cleaner (Jelight) for 3 minutes. The coverslip with gel was carefully removed from the mask and incubated with 10mg/mL EDC and 17mg/mL NHS water solution for 15 minutes, and then coated with 20ug/mL fibronectin (Sigma) and 5ug/mL Alexa546 conjugated fibrinogen (Invitrogen) in HEPES buffer (10mM pH 8.5) for one hour. The photomask was washed with water and then isopropanol.

AFM measurement of micropatterned PAA elasticity

All atomic force microscopy (AFM) measurements were carried out in PBS using a PicoPlus AFM (Agilent Technologies, USA). The spring constant of each cantilever was determined using the thermal noise method (Butt & Jaschke (1995) Nanotechnology 6(1):1-7). Force-indentation profiles were recorded using borosilicate sphere-tipped cantilevers with a radius $R$ = 2.5 $\mu$m (Bioforce Nanoscience, IA, USA) and a spring constant of 60 mN/m. To delimitate insolated and non-insolated zones, topographies of 60 x 60 $\mu$m$^2$ were first imaged in contact mode with 512 x 512 pixels$^2$ at line rates of 0.5 Hz and with the same cantilevers. The sphere probe was then moved above the zone of interest before indentation. The Young's moduli *E* were extracted from the above profiles using the Hertz sphere model for the indentation of a semi-infinite solid. All polyacrylamide samples were assumed to be incompressible (Boudou et al (2006) Biorheology 43:721-728). The expression of the indentation force is thus given by:

$$F = \frac{16E}{9} R^{1/2} \delta^{3/2}$$

where *d* is the indentation. *d* is obtained by subtracting the deflection *d* from the movement of the piezoelectric ceramic ($Dz = z - z_0$) in the *z* direction, where $z_0$ is the contact point, which was determined following the method proposed by Crick and Yin (2007, Biomechanics and Modeling in Mechanobiology 6(3):199-210). For each sample probed, two measurements were taken at five positions. A perfect overlap of two successive indentations performed at each position was obtained, which indicates that the samples were only elastically and not plastically deformed. Young's moduli were calculated by least-square fitting of the experimental force-indentation curves. Measured Young modulus of UV exposed regions was 7.29 +/- 0,42 kPa. Measured Young modulus of non-exposed regions was 6,64 +/- 0,59 kPa.

Traction force measurement

*Bead displacement analysis*

Displacement fields describing the deformation of the PAA substrate are determined from the analysis of fluorescent beads images before and after removal of the adhering cells with trypsin treatment. First, the images are corrected for any translational drift that may have occurred during the experiment by cross-correlating the entire images. The distance of the correlation function maximum from the origin stands for the global translation between the images, which can then be corrected by shifting one image with respect to the other.

The displacement field is obtained in two steps (Sabass et al (2008) Biophys J 94(1):207-220 ; Butler et al (2002) Am J Physiol Cell Physiol 282(3):C595-605). Both images are divided into non-overlapping windows (5.76 $\mu$m squares). In the first step, coarse deformations are computed by cross-correlating each window in one image to the window at the same location in the other image. The result is used to shift one window with respect to the other, so that the global displacement is compensated and only local displacements remain. The second step of the analysis consists in detecting and tracking the beads in the aligned windows, in order to retrieve beads displacements with maximum spatial resolution. Summing the displacements obtained for each step yields the total displacement at the position of the detected beads. The present two-step method provides more reliable results in the case of large displacements or densely packed particles compared to standard particle tracking. Indeed, since part of the total displacement can be corrected following the correlation-based analysis, the cutoff distance in the tracking procedure can be reduced so that different beads would not get mixed up. In addition, compared to pure correlation analysis, the present technique benefits from the intrinsic spatial accuracy of particle tracking since the information relative to each individual bead can potentially be retrieved.

A special procedure is used to evaluate displacements in the area of the adhesive pattern where gel deformation is expected to be largest. Depending on the pattern shape, traction forces may be strongly localized leading to large displacements in very small areas. In this case, failure to track correctly a few beads in such areas would significantly alter the calculated force magnitude. Therefore, the pattern area is divided into smaller windows that are allowed to overlap, before applying the cross-correlation and tracking analysis. Reducing the size of the windows makes it possible to retrieve larger displacements with cross-correlation and, using overlapped windows, we can avoid missing beads close to the windows boundaries. Moreover, bead detection parameters are adjusted independently in the pattern area, since beads are usually less bright under the pattern due to photobleaching during UV irradiation. In this way, the inventors obtain a good tracking efficiency in the pattern region. Since the Fourier-transform traction cytometry (FTTC) method requires that the displacements should be known over a regular rectangular grid, they use a triangle-based linear interpolation to obtain such a field from the beads displacements. The grid spacing is chosen to be 0.72 $\mu$m in x and y directions.

All image processing and analysis were performed using Matlab. The part relative to particle localization and tracking is based on a Matlab package developed by Maria Kilfoil's group (Gao & Kilfoil (2009) Optics Express 17(6):4685-4704) (available at: www.physics.mcgill.ca/~kilfoil/downloads.html).

*Traction force calculation*

To calculate cell-induced traction stress from displacement data, the inventors have used the following assumptions: The substrate is supposed to exhibit a linear elastic behavior and, since the film thickness is large compared to typical displacements and adhesion sizes, it can be approximated as an elastic isotropic half-space so that the Boussinesq Green solution can be applied. Moreover, traction forces are assumed to be tangential to the plane of the substrate. In this case, given an incompressible gel (Poisson ratio close to 0.5), there is no out of plane displacement and the whole problem is two-dimensional. The stress field (local force per unit area) *F(r)* and the displacement field *u(r)* are related by:

$$u_i(r) = \int dr' G_{ij}(r - r') F(r')$$

in which implicit summation are applied with $i,j$=1,2 for two dimensions. $G_{ij}$ is the Boussinesq Green function. To solve the inverse problem of calculating forces from displacements, we applied the Fourier-transform traction cytometry (FTTC) method (Butler et al (2002) Am J Physiol Cell Physiol 282(3):C595-605). The integral is approximated on a discrete computational mesh and the FTTC method takes advantage of the fact that the resulting system of linear equations can be solved more easily in Fourier space where the convolution becomes a simple product. Compared to algorithms that consist in inverting the system of equations in real space, FTTC is computationally much faster and easier to implement, while providing comparable results (Sabass et al, *supra*). Unlike the original work from Butler et al., the inventors used a zero order Tikhonov regularization scheme since the solution of the inverse problem is very sensitive to noise in the displacement data, leading to erratic behavior. Zero-order regularization consists in minimizing $\{|GF - u|^2 + \lambda^2 |F|^2\}$ where a side constraint is added that limits the amplitude of the forces. The regularization parameter $\lambda$ governs the relative importance between the two terms: whether the solution should be in better agreement with displacement data or more regularized. In practice, the inventors perform regularization directly in Fourier space (Sabass et al, *supra*) using the following expression for each wave vector k of the mesh:

$$\tilde{F}(k) = \left[{}'\tilde{G}(k)\tilde{G}(k) + \lambda^2 I\right]^1 \tilde{G}(k)\tilde{u}(k)$$

where $\tilde{\tilde{F}}$ and $\tilde{\tilde{u}}$ are the 2D Fourier transform of the stress and displacement vectors, $\tilde{G}$ is the Fourier transform of the 2D Boussinesq Green function and $I$ is the 2-by-2 identity matrix. A final inverse Fourier transformation is then performed to recover the stress field in real space. The regularization parameter $\lambda$ was adjusted to the lowest value which allows a reasonable solution to be computed. Increasing the regularization parameter has the effect of smoothing out the high spatial frequencies in the stress field. The inventors kept $\lambda$ at small values ($\lambda <\sim10^{-9}$) in order to maintain the best spatial resolution, which is estimated to be about 5 $\mu$m in the present case.

Traction fields induced by cells on equivalent adhesive patterns can be averaged to yield statistically relevant results. Before averaging the calculated stress fields, it is preferable to correct for translation shifts between the images. To this avail, the inventors use the pattern fluorescence images (fibronectin) which are cross-correlated to determine their relative shift. The series of stress images are then aligned according to this criterion before being averaged.

Cell culture

*MCF10A culture*

The culture of MCF10A cells and the generation of $\Delta$CK2$\beta$ cell line were described previously (Deshiere et al (2008) Mol Cell Biochem 316(1-2):107-113). The MCF10A cell expressing ligand inducible Erb2 receptors were obtained from Ariad Pharmaceuticals (Muthuswamy et al (2001) Nat Cell Biol 3(9):785-792). Cells were seeded on micro-patterned substrate at a density of $8x10^4$/cm$^2$. Cells not attaching to the adhesive region on the substrate were washed away 1-2 hours after seeding. All the traction force measurement were performed 6 hours after seeding to ensure full spreading of cell. Substrate relaxation was assessed by detaching cells with trypsin.

To induce Erb2 cell line, AP1510 (Ariad Pharmaceuticals) was added to the culture medium to a final concentration of 1$\mu$M, 48hr before traction force measurement. TGF$\beta$ (R&D systems) was added at 2ng/mL to the culture medium during 48hr before cell plating on micropatterned PAA and traction force measurement.

Blebbistatin(-) (Sigma) at 100$\mu$M was added progressively to the observation chamber to gradually obtain specific final concentration for the drug dose-response experiment. While for the time response experiment, Blebbistatin was added to directly reach a final concentration of 100 $\mu$M. Image acquisition started directly after the drug addition.

*Fixation and immuno-stainings*

Six hours after seeding on micropatterned gel or glass coverslip, cells were first extracted in cytoskeleton buffer (10mM MES, 138mM Kcl, 3mM MgCl, 2mM EGTA, pH6.1) containing 0.5% TritonX-100, then fixed in 4% paraformaldehyde. Fixed samples were washed 3 times in PBS. Afterward, samples were incubated for 1 hour in PBS containing 0.1% Tween, 3% BSA, and 10$\mu$M Phalloidin-FITC (Sigma) to stain actin filaments. On glass cells were immuno labelled with primary antibodies directed against paxilin (BD Tranduction Laboratories) followed by immuno-labelling with secondary Cy3-labelled antibodies (Jackson Immuno Research). All coverslips were stained with Hoechst 33342 (Sigma) to reveal and count cell nuclei. After PBS washing, coverslips were mounted in Mowiol mounting medium.

*Microscopy and Image processing*

Images of fixed cells were taken with a 100X objective (NA=1,35) on an Olympus BX-61 straight microscope, mounted with CDD camera (HQ2, Ropper Scientific) and driven with Metamorph (Molecular Devices).

Live Imaging of beads displacement and micropattern deformation were performed with a 63X objective (NA=1,4) on an inverted 200M Zeiss microscope, mounted with CDD camera (HQ2, Ropper Scientific) and driven with Metamorph (Molecular Devices). Temperature and CO2 control were ensured by the Cube and the Box from LIS Imaging.

All the acquired images were processed by ImageJ (http://rsb.info.nih.gov/ij/). Averaged fluorescent staining images were automatically aligned using micropattern images by a custom written plugins. Pattern detection and length measurement were done automatically by custom written macro routines.

## Claims

1. Process of fabrication of a soft substrate, comprising the steps of :

   (a) producing a polymer between a base layer and a transparency mask comprising at least one transparent area and optionally at least one non-transparent area,
   (b) exposing the polymer to deep UV through the mask,
   (c) detaching the mask from the polymer,
   (d) contacting the polymer with a a biomolecule, and
   (e) optionally washing away the excess of biomolecule,

   wherein the soft substrate has a Young's modulus in the range of about 0.1 to about 100 kPa,
   wherein the polymerization is performed *in situ,* and
   wherein the biomolecule is a protein, a carbohydrate, a lipid, a nucleic acid, or a combination thereof.

2. Process according to claim 1, wherein the base layer is silanized glass.

3. Process according to claim 1 or 2, wherein the polymer is polyacrylamide.

4. Process according to anyone of the previous claims, wherein polymerization reagents implemented for the production of the polymer of step (a) are added as a solution onto the mask and then the base layer is applied thereon.

5. Process according to anyone of the previous claims, wherein the mask is a quartz plate coated with a chromium layer, where the chromium layer is absent on specific areas.

6. Process according to anyone of the previous claims, wherein the mask comprises between 10 and 50 000 transparent areas/cm$^2$.

7. Process according to anyone of the previous claims, wherein the biomolecule is a protein, in particular chosen among fluorescent proteins, extracellular matrix proteins, and proteins allowing cells to fix thereto.

8. Process according to claim 7, wherein the protein is fibronectin.

9. A soft substrate obtainable by the process according to anyone of the previous claims.

10. A use of a soft substrate according to claim 9 for cell culture.

11. A method for assaying a cellular activity or property of a cell, comprising providing a soft substrate according to claim 9, exposing said substrate to at least one cell for a period of time sufficient to allow the cell(s) to bind to the soft substrate, and measuring the cellular activity or property of said cell.

12. A method for identifying a molecule modulating a cellular activity or property of a cell comprising providing a soft substrate according to claim 9, exposing said substrate to at least one cell for a period of time sufficient to allow the cell(s) to bind to the soft substrate, and measuring the cellular activity or property of said cell in presence and in absence of the molecule, and determining the effect of the molecule on the cellular activity or property, wherein a modulation of the cellular activity or property indicates that the molecule is capable of modulating the cellular activity or property of the cell.

## Patentansprüche

1. Herstellungsverfahren eines weichen Substrats umfassend die Schritte:

   (a) Herstellen eines Polymers zwischen einer Basisschicht und einer Transparenzmaske umfassend mindestens einen transparenten Bereich und optional mindestens einen nicht transparenten Bereich,
   (b) Aussetzen des Polymers einer tiefen UV durch die Maske,
   (c) Ablösen der Maske vom Polymer,
   (d) In Kontakt bringen des Polymers mit einem Biomolekül, und
   (e) optionales Auswaschen des Überschusses an Biomolekül, wobei das weiche Substrat einen Young Modulus in dem Bereich von etwa 0,1 bis etwa 100 kPa hat,

   wobei die Polymerisierung *in situ* durchgeführt wird und wobei das Biomolekül ein Protein, ein Kohlenhydrat, ein Lipid, eine Nukleinsäure oder eine Kombination daraus ist.

2. Verfahren nach Anspruch 1, wobei die Basisschicht silanisiertes Glas ist.

3. Verfahren nach Anspruch 1 oder 2, wobei das Polymer Polyacrylamid ist.

4. Verfahren nach einem der vorhergehenden Ansprüche, wobei Polymerisierungsreagenzien, die für die Herstellung des Polymers aus Schritt (a) implementiert sind, als eine Lösung auf die Maske gegeben werden und dann darauf die Basisschicht appliziert wird.

5. Verfahren nach einem der vorhergehenden Ansprüche, wobei die Maske eine Quartzplatte ist, die mit einer Chromschicht beschichtet ist, wobei die Chromschicht an bestimmten Bereichen nicht vorhanden ist.

6. Verfahren nach einem der vorhergehenden Ansprüche, wobei die Maske zwischen 10 und 50000 transparenten Bereichen/cm2 umfasst.

7. Verfahren nach einem der vorhergehenden Ansprüche, wobei das Biomolekül ein Protein ist, bevorzugt ausgewählt unter fluoreszierenden Proteinen, extrazellulären Matrix Proteinen und Proteinen, die Zellen erlauben daran zu haften.

8. Verfahren nach Anspruch 7, wobei das Protein Fibronectin ist.

9. Ein weiches Substrat erhältlich durch das Verfahren nach einem der vorhergehenden Ansprüche.

10. Eine Verwendung eines weichen Substrats nach Anspruch 9 zur Zellkultur.

11. Ein Verfahren zur Untersuchung einer zellulären Aktivität oder Eigenschaft einer Zelle umfassend das Bereitstellen eines weichen Substrats nach Anspruch 9, Aussetzen des Substrats mindestens einer Zelle für eine ausreichende Zeitdauer um der/den Zelle(n) zu erlauben, an das weiche Substrat zu binden und Messen der zellulären Aktivität oder Eigenschaft der Zelle.

12. Ein Verfahren zur Identifizierung eines Moleküls, das eine zelluläre Aktivität oder Eigenschaft einer Zelle moduliert, umfassend das Bereitstellen eines weichen Substrats nach Anspruch 9, Aussetzen des Substrats mindestens einer Zelle für eine ausreichende Zeitdauer um der/den Zelle(n) zu erlauben, an das weiche Substrat zu binden und Messen der zellulären Aktivität oder Eigenschaft der Zelle in Anwesenheit und Abwesenheit des Moleküls, und Bestimmen des Effekts des Moleküls auf die zelluläre Aktivität oder Eigenschaft, wobei eine Modulation der zellulären Aktivität oder Eigenschaft anzeigt, dass das Molekül fähig ist, die zelluläre Aktivität oder Eigenschaft der Zelle zu modulieren.

**Revendications**

1. Procédé de fabrication d'un substrat mou, comprenant les étapes de :

(a) production d'un polymère entre une couche de base et un masque transparent comprenant au moins une zone transparente et de façon optionnelle au moins une zone non-transparente,
(b) exposition du polymère à l'UV profond à travers le masque,
(c) détachement du masque du polymère,
(d) mise en contact du polymère avec une biomolécule, et
(e) de façon optionnelle, lavage de l'excès de biomolécule,

dans lequel le substrat mou a un module d'Young dans la plage d'environ 0.1 à environ 100 kPa, dans lequel la polymérisation est réalisée *in situ*, et dans lequel la biomolécule est une protéine, un glucide, un lipide, un acide nucléique, ou une combinaison de ceux-ci.

2. Procédé selon la revendication 1, dans lequel la couche de base est du verre silanisé.

3. Procédé selon la revendication 1 ou 2, dans lequel le polymère est du polyacrylamide.

4. Procédé selon l'une quelconque des revendications précédentes, dans lequel les réactifs de polymérisation implémentés pour la production du polymère de l'étape (a) sont ajoutés sous forme de solution sur le masque et ensuite la couche de base est appliquée sur celui-ci.

5. Procédé selon l'une quelconque des revendications précédentes, dans lequel le masque est une plaque de quartz recouverte avec une couche de chrome, où la couche de chrome est absente au niveau de zones spécifiques.

6. Procédé selon l'une quelconque des revendications précédentes, dans lequel le masque comprend entre 10 et 50 000 zones transparentes/cm$^2$.

7. Procédé selon l'une quelconque des revendications précédentes, dans lequel la biomolécule est une protéine, en particulier choisie parmi des protéines fluorescentes, des protéines de la matrice extracellulaire et des protéines permettant aux cellules de s'y fixer.

8. Procédé selon la revendication 7, dans lequel la protéine est une fibronectine.

9. Substrat mou pouvant être obtenu par le procédé selon l'une quelconque des revendications précédentes.

10. Utilisation d'un substrat mou selon la revendication 9 pour la culture cellulaire.

11. Méthode pour l'analyse d'une activité cellulaire ou d'une propriété d'une cellule, comprenant la fourniture d'un substrat mou selon la revendication 9, l'exposition dudit substrat à au moins une cellule pendant une période de temps suffisante pour permettre à la (aux) cellule(s) de s'attacher au substrat mou, et la mesure de l'activité cellulaire ou de la propriété de ladite cellule.

12. Méthode pour l'identification d'une molécule modulant une activité cellulaire ou une propriété d'une cellule comprenant la fourniture d'un substrat mou selon la revendication 9, l'exposition dudit substrat à au moins une cellule pour une période de temps suffisante pour permettre à la (aux) cellule(s) de s'attacher au substrat mou, et la mesure de l'activité cellulaire ou de la propriété de ladite cellule en présence et en absence de la molécule, et la détermination de l'effet de la molécule sur l'activité cellulaire ou la propriété, dans laquelle une modulation de l'activité cellulaire ou de la propriété indique que la molécule est capable de moduler l'activité cellulaire ou la propriété de la cellule.

FIGURE 1A

FIGURE 1B

PA + sulfo-SANPAH     PA + deep UV     Petri dish

FIGURE 1C

**FIGURE 1**

FIGURE 2A

FIGURE 2B

FIGURE 2C

FIGURE 2D

no pattern  disc  pacman  crossbow

FIBRONECTIN

F-ACTIN

**FIGURE 3A**

embedded beads

**FIGURE 3B**

no pattern  disc  pacman  crossbow

TRACTION
IN SINGLE CELLS (Pa)

**FIGURE 3C**

AVERAGED
TRACTION (Pa)

n=20  n=30  n=9  n=35

**FIGURE 3D**

**FIGURE 4A**

**FIGURE 4B**

**FIGURE 4C**

**FIGURE 4D**

**FIGURE 4E**

**FIGURE 4F**

## Dose-response

**FIGURE 5A**

**FIGURE 5B**

**FIGURE 5C**

**EP 2 492 340 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010011407 A
- WO 2005026313 A
- WO 2010046459 A

### Non-patent literature cited in the description

- **DAMLJANOVIC V et al.** *Biotechniques,* 2005, vol. 39, 847-851
- **PARKER et al.** *FASEB J,* 2002, vol. 16, 1195-1204
- **THERY et al.** *Cell Motil Cytoskeleton,* 2006, vol. 63, 341-355
- **THERY et al.** *Proc Natl Acad Sci USA,* 2006, vol. 103, 19771-19776
- **WANG et al.** *Cell Motil Cytoskeleton,* 2002, vol. 52, 91-106
- **ENGLER AJ et al.** *J Cell Biol,* 2004, vol. 166, 877-887
- **LIU et al.** *FASEB J,* 2002, vol. 16, 1195-1204
- **RUIZ et al.** *Stem Cells,* 2008, vol. 26, 2921-2927
- **TAN et al.** *Proc Natl Acad Sci USA,* 2003, vol. 100, 1484-1489
- **FU et al.** *Nat Methods,* 2010, vol. 7, 733-736
- **DEMBO ; WANG.** *Biophys J,* 1999, vol. 76, 2307-2316
- **DU ROURE et al.** *Proc Natl Acad Sci USA,* 2005, vol. 102, 2390-2395
- **ENGLER et al.** *Cell,* 2004, vol. 126, 677-689
- **YEUNG et al.** *Cell Motility and the Cytoskeleton,* 2005, vol. 60, 24-34
- **MARGANSKI et al.** *Methods Enzymol,* 2003, vol. 361, 197-211
- **THERY.** *J Cell Science,* 2010, 123
- **SABASS et al.** *Biophys J,* 2008, vol. 94 (1), 207-220
- **BUTLER et al.** *Am J Physiol Cell Physiol,* 2002, vol. 282 (3), C595-605
- **MITROSSILIS D et al.** *Proc Natl Acad Sci U S A,* 2009, vol. 106 (43), 18243-18248
- **TAN JL et al.** *Proc Natl Acad Sci U S A,* 2003, vol. 100 (4), 1484-1489
- **BUTT ; JASCHKE.** *Nanotechnology,* 1995, vol. 6 (1), 1-7
- **BOUDOU et al.** *Biorheology,* 2006, vol. 43, 721-728
- **CRICK ; YIN.** *Biomechanics and Modeling in Mechanobiology,* 2007, vol. 6 (3), 199-210
- **GAO ; KILFOIL.** *Optics Express,* 2009, vol. 17 (6), 4685-4704, www.physics.mcgill.ca/~kilfoil/downloads.html
- **DESHIERE et al.** *Mol Cell Biochem,* 2008, vol. 316 (1-2), 107-113
- **MUTHUSWAMY et al.** *Nat Cell Biol,* 2001, vol. 3 (9), 785-792